# EUROPÄISCHE PATENTSCHRIFT

(11) **EP 0 968 273 B1**
(45) Veröffentlichungstag und Bekanntmachung des Hinweises auf die Patenterteilung: **15.05.2002**
(21) Anmeldenummer: 98929218.0
(22) Anmeldetag: 09.04.1998
(51) Int. Cl.: C12M 1/00, A01G 33/00

(54) **ANLAGE ZUR DURCHFÜHRUNG VON PHOTOCHEMISCHEN UND PHOTOKATALYTISCHEN REAKTIONEN UND PHOTOINDUZIERBAREN PROZESSEN**
INSTALLATION FOR CARRYING OUT PHOTOCHEMICAL AND PHOTOCATALYTIC REACTIONS AND PHOTOINDUCED PROCESSES
INSTALLATION POUR EFFECTUER DES REACTIONS PHOTOCHIMIQUES ET PHOTOCATALYTIQUES ET DES PROCESSUS PHOTOINDUCTIFS

(30) Priorität: 10.04.1997 DE 19714819; 10.04.1997 DE 29706379 U; 31.03.1998 DE 19814424
(43) Veröffentlichungstag der Anmeldung: 05.01.2000
(73) Patentinhaber: Bioprodukte Prof. Steinberg GmbH, 06217 Merseburg (DE)
(72) Erfinder: BRONESKE, Jürgen, D-14943 Felgentreu (DE); PULZ, Otto, D-14558 Bergholz-Rehbrücke (DE); FRANKE, Horst, D-14558 Bergholz-Rehbrücke (DE); DÖBEL, Katrin, D-14558 Bergholz-Rehbrücke (DE); OEHLMANN, Hans-Ulrich, D-31141 Hildesheim (DE); UPHOFF, Rainer, D-38122 Braunschweig (DE)
(74) Vertreter: Köckeritz, Günter
(86) Internationale Anmeldenummer: DE9801012
(87) Internationale Veröffentlichungsnummer: WO9845405

(56) Entgegenhaltungen:
- EP-A- 0 261 872
- DE-A- 3 923 251
- DE-U- 29 607 285
- DE-U- 29 706 379
- US-A- 2 732 663
- US-A- 3 865 691

## Beschreibung

Die Erfindung betrifft eine Anlage zur Durchführung von photochemischen und photokatalytischen Reaktionen und photoinduzierbaren Prozessen, insbesondere zur Kultivation von phototrophen Organismen und Zellkulturen, z. B. Mikroalgen.

Bekannte Vorrichtungen zur Kultivation von phototrophen Organismen und Zellkulturen, wie z. B. Mikroalgen, sind entweder als offene, unter freiem Himmel angeordnete Becken ausgebildet oder werden ausschließlich mit Kunstlicht betrieben, wobei das Kulturmedium in transparenten Rohren geführt wird. Vorrichtungen mit behälterförmigen Reaktoren sind meistens noch mit Mitteln zur Umwälzung des Kulturmediums zur Erreichung einer intensiven Belichtung ausgerüstet, wodurch der konstruktive Aufwand die damit verbundenen Kosten erhöht.
Bei den offenen Becken besteht ständig die Gefahr der Kontamination, so daß eine Reinzucht nicht möglich ist. Auch die Temperaturführung solcher Anlagen gestaltet sich unter den hier herrschenden klimatischen Bedingungen als sehr schwierig.

Aus der US-PS 4,473,970 ist eine mit Kunstlicht betreibbare Vorrichtung bekannt, in der das Kulturmedium in transparenten Rohren geführt wird. Nachteilig bei dieser Vorrichtung sind vor allem energetische Aspekte bezüglich der Pumpen- und Lichtleistung.
Weiterhin sind Vorrichtungen bekannt, die aus behälterförmigen Reaktoren bestehen, in die das Licht über Lichtleiter eingeleitet wird. Das Licht wird über eine der Sonne nachführbare Fresnel-Linse und/oder über eine Kunstlichtquelle über die gleichen Lichtleiter in das Kulturmedium geleitet. Solche Anlagen sind sowohl von der Herstellung als auch im Betrieb sehr kostenintensiv.
In der US - PS 4,555,864 wird eine Einrichtung zur Kultivation von Chlorella-Algen beschrieben, bei der Lichtleiter in einem Kultivationsgefäß direkt in das Kulturmedium eintauchen, wobei diese von oben her über schräg gestellte Spiegel mit Lichtenergie versorgt werden. Diese Einrichtung ist ebenfalls sehr kostenintensiv und konstruktiv aufwendig.
Die DE 41 34 813 A1 beschreibt ebenfalls eine Einrichtung zur Kultivation von phototrophen Mikroorganismen, die unter Einsatz von Sonnen- und/oder Kunstlicht betreibbar ist und von einem Kulturmedium durchströmt wird. Diese Einrichtung ist gekennzeichnet durch eine beliebige Anzahl von Stegen, die jeweils im gleichen Abstand voneinander zwischen zwei planparallelen, transparenten Platten flüssigkeitsdicht angeordnet sind und Kammern bilden. Jeweils am Ende eines Steges sind Verbindungsöffnungen zur benachbarten Kammer vorgesehen, wobei die Verbindungsöffnungen jeweils benachbarter Stege sich an den entgegengesetzten Enden befinden, wodurch ein mäanderlinienförmig durchströmbarer Kanal gebildet wird, der an seinen Stirnseiten dicht verschlossen ist und an seinem Anfang und Ende je einen Anschlußstutzen für den Gasaustausch aufweist. Diese Einrichtung ist für einen großtechnischen Einsatz schon aus energetischen Gründen wegen der zu erwartendenen hohen Pumpenleistung zur Überwindung des Strömungswiderstandes der mäanderlinienförmig durchströmbaren Kanäle und des hohen Herstellungsaufwandes einer mit derartigen Stegen versehenen Platte ungeeignet.

Die Veröffentlichung nach Spektorova, L.;Creswell, R.L.; Vaughan, D.(1997): Closed tubular cultivators, World auqaculture, 6,S.39-43 faßt den Stand der röhrenförmigen Bioreaktoren, welche vorwiegend biologisch aktive Substanzen für die Medizinische und Kosmetikindustrie produzieren, zusammen. Ein horizontal angeordneter Glasrohrbioreaktor mit 14 m³ Kulturvolumen wurde dabei in Usbekistan installiert. Der Glasrohrbioreaktor produziert dabei eine Biomasse (Chlorella vulgaris) von ca. 2 t/Jahr mit einer maximalen Produktivität von 43 g/m^{2x}d.
Sehr problematisch ist dabei zum einen die Suspensionsverteilung in den einzelnen Glasrohren, die Temperierung des Systems und die sehr hohe Sauerstoffkonzentration in der Suspension. Zudem erfolgt die Montage der einzelnen horizontalen Glasrohre mit Flanschverbindungen, welche relativ hohe Kosten verursachen und Spalträume für abgestorbene Biomasse bzw. Bakterien bilden.

Nach den Veröffentlichungen von Pulz, O. (1992a): Open-air and semi-closed cultivation systems for the mass cultivation of microalgae. 1^{st} Asia-Pacific Conference Algal Biotechnol.Kuala Lumpur, Malaysia und Pulz, O. et al. (1992b): Technische Aspekte der Kultivierung autotropher Mikroorganismen, Forschungsbericht zum BMFT-Projekt Nr. 322-4003-0319894A, werden fast geschlossene tubuläre Glasrohrfermenter (Rohr d = 2,5 cm) betrieben, wobei eine Kreiselpumpe 50 I Algensuspension schlaufenförmig um die Lichtquelle pumpt. Nimmt man die Glasröhrenmantelfläche als Berechnungsgrundlage, so übertrifft die flächenbezogene Zuwachsrate mit 14 g m⁻² d⁻¹ nicht die bereits erwähnten Produktivitäten offener Systeme.

Nach der Veröffentlichung von Miyamoto et al. (1988): Vertical tubular reactor for microalgae cultivation, Biotechnology-Letters, 10(10), 703-708, wird die sehr kostengünstige Variante, Mikroalgen in vertikal stehenden Glasrohren mit d = 5 cm (Oberflächen-Nolumenverhältnis von 80 m⁻¹) und 2 m Höhe zu kultivieren genutzt, die für die Fluoreszenzlampenproduktion in Massen hergestellt werden. Durch eine Vielzahl paralleler Kolben läßt sich ein Scale up mühelos erreichen, jedoch erwies sich die Ernte als schwierig und bei Naturbeleuchtung ergaben sich erhebliche Hitzeprobleme. Die maximalen Produktivitäten beliefen sich je nach kultivierter Art von 0.3 g I⁻¹ d⁻¹ mit dem Cyanobakterium Nostoc bis 0.6 g I⁻¹ d⁻¹ bei der Grünalge Monoraphidium.

In der Veröffentlichung von Jüttner, F. (1977) Thirty liter tower-type pilot plant for the mass cultivation of light- and motion-sensitive planktonic algae, Biotechnology and Bioengineering, 19, 1679-1687, wird das Hitzeproblem gelöst, indem er einen weiteren inneren Tubus installierte, der zum einen Kühlflüssigkeit enthielt und zum anderen das Dunkelvolumen der Suspension verminderte. Dadurch gelang ihm eine weitere Wachstumsbeschleunigung um den Faktor 1.5. Seine technisch aufwendige und bedingt Scale up-fähige Idee war jedoch Vorbild für die Konstruktion einiger Freilandröhrenanlagen mit Gegenstromkühlung (z. B. FR-PS 2 564 854).

DE-U-297 06 379 offenbart einen Bioreaktor zur Durchführung von photochemischen und photokatalytischen Reaktionen, die eine Plattenmodulanlage als phothosyntetisch und hydrodynamisch aktives Dünnschichtsystem, ein Vor- und Rücklaufsystem, Gaszufuhr- und Gasabfuhrelemente, ein Konditionierbehälter, ein Wärmetauscher, eine Systempumpe und eine Meßstrecke umfaßt, EP-A-0 261 872 offenbart eine Anlage zur Produktion von Mikroorganismen, die aus einem Bioreaktor, einer Erntevorrichtung, einer Nährlösungsversorgungs-einrichtung und einer Nährlösungsaufbereitungsanlage besteht. Die Anlage arbeitet nicht nach dem "Dünnschichtprinzip".

Kennzeichnend für alle zylindrischen Reaktoren ist eine hohe Leuchtdichte in bezug auf ein kleines Oberflächen-/Volumenverhältnis, so daß, um ausreichender Belichtung der Algenzelle Genüge zu leisten bzw. Photoinhibition zu vermeiden, hohe Strömungsgeschwindigkeiten notwendig sind. Starke Turbulenzen jedoch erzeugen dementsprechende Scherkraftbelastungen, welche sich bei den meisten Mikroalgenarten kontraproduktiv auswirken. Zudem wäre eine effektive Ausleuchtung größerer Anlagen nur sehr schwer zu realisieren bzw. nur unter einem erheblichen technischen Aufwand möglich.

Der vorliegenden Erfindung liegt die Aufgabe zugrunde, eine Anlage zur effizienten Produktion von Mikroorganismen bereitzustellen. Bekannte Einrichtungen nach dem "Dünnschichtprinzip" zur effizienten Produktion von Mikroorganismen, insbesondere von phototrophen Mikroorganismen hinsichtlich des Kultivations- und Ernteprozesses sollen dahingehend verbessert werden, daß im gesamten Jahresverlauf unter optimaler Ausnutzung des natürlichen Sonnenlichts und optimaler Konditionierung der Kultursuspension, insbesondere der energetischen und stofflichen Austauschprozesse sowie der hydrodynamischen Strömungsverhältnisse, eine maximale Produktion von Mikroorganismen realisiert und gleichzeitig im Zusammenwirken der peripheren Ausrüstung ein optimales Betriebsergebnis gewährleistet wird.

Erfindungsgemäß wird diese Aufgabe durch die Merkmale des Anspruches 1 gelöst. Vorteilhafte Ausbildungen der Erfindung sind in den Ansprüchen 2 bis 13 enthalten.

Die gemäß der Erfindung vorgestellte Einrichtung zur effizienten Produktion von Mikroorganismen enthält einen Bioreaktor, eine Emtevorrichtung, eine Nährlösungs- bzw. Stammlösungsversorgungsvorrichtung und eine Nährlösungsaufbereitungsanlage.

Der Bioreaktor besteht aus photosynthetisch und hydrodynamisch (turbulär) aktiven Dünnschichtsystemen, einem Vor- und Rücklaufsystem, einem Konditionierbehälter, einem Wärmetauscher, einer Systempumpe, einer Meßstrecke, einem Mittel zur quantitativen und qualitativen Veränderung der Gefäßbelichtung und einem Mittel zur Gaszu- bzw. -ableitung, wobei erfindungsgemäß das photosynthetisch und hydrodynamisch aktive Dünnschichtsystem aus einer Vielzahl von übereinanderliegenden und parallel zueinander verlaufenden durchgehenden Röhren oder Schläuchen besteht, deren Eintritts- und Austrittsseiten über Anschlußstücke und einen querschnittsreduzierten Verbinder mit einem Modulvor- bzw. -rücklauf verbunden sind, wobei die einzelnen Verbinder für die übereinanderliegenden Dünnschichtgefäße jeweils eine Querschnittsfläche aufweisen, die ein gleichmäßiges Strömungsprofil über alle Dünnschichtgefäße ermöglicht. Nach einem besonderen Merkmal der Erfindung weist der querschnittsreduzierte Verbinder in Fließrichtung des Modulvorlaufs eine Querschnittserweiterung und/oder in Fließrichtung des Modulrücklaufs eine Querschnittsreduzierung auf.

Nach einem weiteren Merkmal der Erfindung besteht der Modulvorlauf bzw. der Modulrücklauf aus einem Rohr.

Es ist dabei vorteilhaft, wenn der Modulvorlauf in Fließrichtung einen geringeren Durchmesser und der Modulrücklauf in Fließrichtung einen größeren Durchmesser besitzt.
Nach einem besonderen Merkmal der Erfindung besitzt das transparente Dünnschichtgefäß Konditionierungsturbulatoren, welche auch bei geringer Fließgeschwindigkeit turbulente Strömungsverhältnisse gewährleisten, das Oberflächen-/Grundflächenverhältnis erhöhen, das einfallende Licht optimal im transparenten Dünnschichtgefäß verteilen und eventuell auftretende Immobilisierungen an den Gefäßoberflächen durch eine effizientere Reinigungswirkung von Reinigungskörpern in der Suspension ermöglichen, wobei die Konditionierungsturbulatoren derart gestaltet sind, daß keine hydraulischen Totäume im transparenten Dünnschichtgefäß entstehen.

Nach einem weiteren Merkmal der Erfindung besteht das transparente Dünnschichtgefäß aus mindestens einem Glasrohrmodul mit übereinanderliegenden Glasrohren, welche die Konditionierungsturbulatoren bereits bei der Herstellung der Glasrohre erhalten.
Nach einer bevorzugten Ausführungsform der Erfindung setzt sich die Gesamtlänge der Glasrohre aus einzelnen Rohren zusammen, welche hydraulisch ohne Unterbrechungen zusammenverklebt oder -verschweißt sind.

Es ist eine vorteilhafte Ausführungsform der Erfindung, daß die Mittel zur quantitativen und qualitativen Veränderung der Gefäßbelichtung aus UV-fluoreszierendem und/oder reflektierendem und/oder antireflektierendem und/oder transparentem Material bestehen, welches die zur Verfügung stehende Strahlungsmenge im photosynthetisch aktiven Bereich für die Belichtung der Mikroorganismen in den Dünnschichtgefäßen erhöht.
Es ist dabei vorteilhaft, daß die Oberfläche und/oder das Wandmaterial des transparenten Dünnschichtgefäßes aus UV-fluoreszierendem und/oder antireflektierendem Material besteht, welches die zur Verfügung stehende Strahlungsmenge im photosynthetisch aktiven Bereich für die Belichtung der Mikroorganismen in den Dünnschichtgefäßen erhöht und/oder zwischen den Dünnschichtgefäßen UV-fluoreszierendes und transparentes Material und/oder unter den Dünnschichtgefäßen UV-fluoreszierendes Material vorhanden ist.

Nach einem weiteren Merkmal der Erfindung bestehen die Mittel zur quantitativen und qualitativen Veränderung der Gefäßbelichtung aus IR-reflektierendem und/oder transparentem Material und werden an der Oberfläche bzw. im Wandmaterial des transparenten Dünnschichtgefäßes eingebunden, wobei eine Erhöhung der Suspensionstemperatur vermindert wird und eine Strahlungskühlung der Kultursuspension durch das Wandmaterial des transparenten Dünnschichtgefäßes gewährleistet wird.

Es ist im Sinne der Erfindung, daß die Mittel zur Gaszu- bzw. -ableitung aus einem Rücklaufsammler, welcher im oder auf dem Boden angeordnet ist und temperiert wird, einer CO₂-Einbindung in die Kultursuspension, einen O₂-Austrag aus der Kultursuspension und einem Gasverteiler im Rücklaufsammler bestehen.
Es ist dabei vorteilhaft, den Mitteln zur Gaszu- bzw. -ableitung Latentwärmespeichermaterial zuzuordnen.

Eine besonders bevorzugte Ausführungsform der Erfindung beinhaltet, daß der Konditionierbehälter, der Wärrnetauscher und die Mittel zur Gaszu- bzw. - ableitung miteinander verbunden sind und gleichzeitig im Komplex wirken.

Es ist dabei vorteilhaft, daß die Mittel zur Gaszu- bzw. -ableitung, insbesondere der Rücklaufsammler, gleichzeitig einen Teil des Wärmetauschers und/oder des Konditionierbehälters bilden und somit Synergieeffekte hinsichtlich einer optimalen Konditionierung der Kultursuspension erzielt werden.

Die Erfindung ist weiterhin ausgestaltet mit einer Erntevorrichtung, welche aus einem Erntebehälter, einer Erntepumpe, einem Separator und einem Biomassebehälter besteht.

Die Erfindung ist zudem ausgestaltet mit einer Nährlösungs- bzw.
Stammlösungsversorgungsvorrichtung, welche aus einer Nährlösungspumpe, einem Stammlösungsbehälter mit einem Rührwerk und einem Nährlösungsbehälter besteht.

Nach einem besonderen Merkmal der Erfindung weist die Emtevorrichtung und/oder Nährlösungs- bzw. Stammlösungsversorgungsvorrichtung eine Nährlösungsaufbereitungsvorrichtung auf, wodurch die abseparierte Kultursuspension aus der Erntevorrichtung als Nährlösung und/oder die Nährbzw. Stammlösung aus der Nährlösungs- bzw. Stammlösungsversorgungsvorrichtung in den Bioreaktor kontaminationsfrei gelangen kann.

Es ist im Sinne der Erfindung, daß die Nährlösungsaufbereitungsvorrichtung aus einem mechanischen Vorfilter und einem Aktivkohlefilter besteht, welcher kontaminierende Stoffe ausfiltert und Nährstoffe ungehindert durchströmen läßt.

Eine besonders bevorzugte Ausführungsform der Erfindung beinhaltet, daß die Erntevorrichtung, die Nährlösungs- bzw. Stammlösungsversorgungsvorrichtung und die Nährlösungsaufbereitungsvorrichtung miteinander verbunden sind und gleichzeitig im Komplex wirken.
Nachfolgend soll die Erfindung anhand von Ausführungsbeispielen näher erläutert werden.
In den Zeichnungen zeigen die
- Fig. 1: die schematische Darstellung einer Ausführungsvariante der erfindungsgemäßen Anlage,
- Fig. 2: ein Glasrohrmodul mit Vor- und Rücklauf sowie Konditionsierungsturbulatoren,
- Fig. 3: ein Glasrohrmodul (Seitenansicht) mit Rücklaufsammler und Mittel zur quantitativen und qualitativen Veränderung der Gefäßbelichtung.

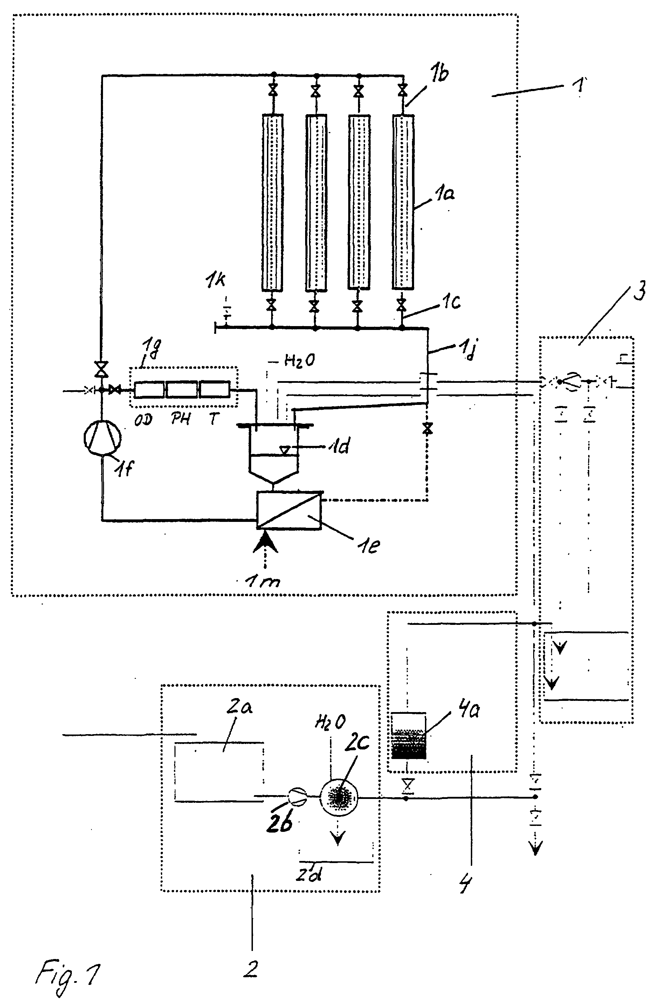

Die Figur 1 zeigt in einer schematischen Darstellung eine Ausführungsvariante der erfindungsgemäßen Anlage zur effizienten Produktion von phototrophen Organismen und Zellkulturen, insbesondere von Mikroalgen, unter Lichteinwirkung.

Die Anlage setzt sich zusammen aus einem Bioreaktor 1, der Erntevorrichtung 2, einer Nährlösungs- bzw. Stammlösungsversorgungseinrichtung 3 und einer Nährlösungsaufbereitungsvorrichtung 4.

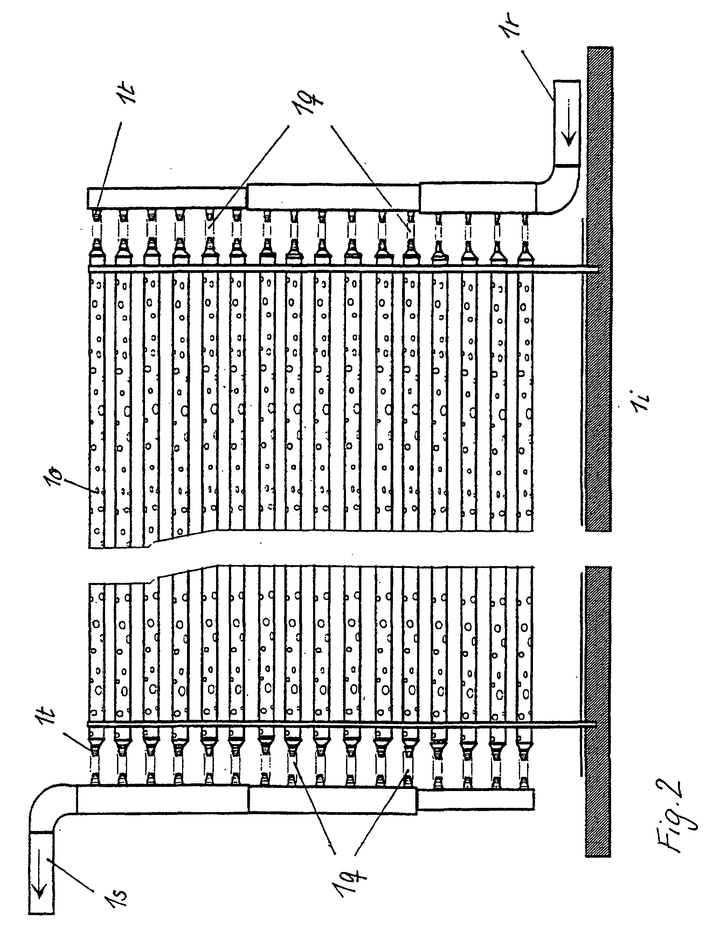

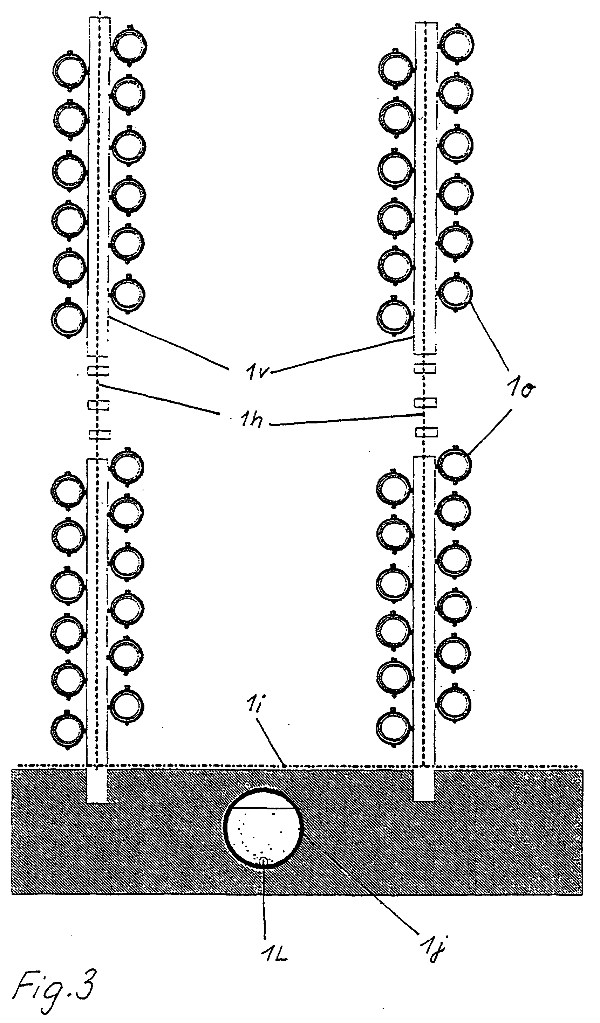

Gemäß der Ausführungsvariante in Figur 1 wird zunächst durch Beimpfen einer Nährlösung mit Mikroorganismen eine Kultursuspension bzw. Stammlösung hergestellt. Die Vorrichtung 3 besteht im wesentlichen aus einem Nährlösungsbehälter, einer Nährlösungspumpe und einem Stammlösungsbehälter mit Rührwerk. Von hier aus wird die Nährlösung zum Bioreaktor 1 geleitet. Der Bioreaktor 1 besteht im vorliegenden Beispiel im wesentlichen aus vier Modulen eines photosynthetisch und hydrodynamisch aktiven Dünnschichtsystems 1a, die das Reaktorgefäß bilden, einem Vor- und Rücklaufsystem 1b bzw. 1c, einem Konditionierbehälter 1d, einem Wärmetauscher 1e, einer Systempumpe 1f, einer Meßstrecke 1g, Mitteln zur quantitativen und qualitativen Veränderung der Reaktorgefäßbelichtung 1h und 1i (siehe Figuren 2 und 3) und Mitteln zur Gaszu- bzw. -ableitung. Die Mittel zur Gaszu- bzw. -ableitung bestehen im beschriebenen Beispiel aus einem Rücklaufsammler 1j, welcher hier im Boden der Anlage angeordnet ist, einer Vorrichtung zur CO₂-Einbindung (nicht dargestellt) in die Kultursuspension im Rücklaufsammler 1j, einem O₂-Austrag 1k und einem Gasverteiler 1I (Figur 3).

In dem Rücklaufsammler 1j befindet sich ein Gasverteiler 1I, wobei der Gasverteiler 1I über die gesamte Länge des Rücklaufsammlers 1j ein Abgas mit einer CO₂-Konzentration von 25 % in die Kultursuspension dosiert. Im Heizfall besitzt das Abgas eine maximale Temperatur von 38 °C und erwärmt die Kultursuspension über den Wärmetauscher 1e.
Der Wärmetauscher 1e gewährleistet dabei eine optimale Abgas- bzw. Suspensionstemperatur.

Zur Erhöhung der Wärmeübertragung wird der Wärmetauscher 1e in der Ansaugleitung der Systempumpe 1f installiert. Die Konditionierung des C02-enthaltenden Abgases erfolgt in Abhängigkeit von pH-, Temperatur- und optischen Dichtewerten der Kultursuspension, welche in der Meßstrecke 1g vor der Systempumpe 1f erfaßt werden. Die Messung, Steuerung, Regelung und Speicherung von Zustandsgrößen der Kultursuspension und des CO₂enthaltenden Abgases erfolgt dabei über eine zentrale Meß-, Steuer-, Regelund Speichereinheit (nicht dargestellt). Die konditionierte Kultursuspension wird mit Hilfe der Systempumpe 1f über einen Pumpenvorlauf 1m zum photosynthetisch und hydrodynamisch aktiven Dünnschichtsystem 1a des Bioreaktors 1 geführt. Das Dünnschichtsystem 1a besteht nach diesem Beispiel aus vier Modulen, wobei jedes Modul aus einer Vielzahl von zueinander beabstandeten, übereinanderliegenden und parallel zueinander verlaufenden Dünnschichtgefäßen (Glasrohren) 10 besteht, deren Eintritts- und Austrittsseiten über Anschlußstücke 1p und Verbinder 1q mit einem Modulvorlauf 1r bzw. -rücklauf 1s verbunden sind. Die einzelnen Verbinder 1q weisen dabei jeweils eine Querschnittsfläche auf, die ein gleichmäßiges Strömungsprofil über alle Glasrohre 1o ermöglicht. Dabei nimmt die Querschnittsfläche der Verbinder 1q in Fließrichtung des Modulvorlaufs 1r zu, während die Querschnittsfläche der Verbinder 1q des Modulrücklaufs 1s in Fließrichtung abnimmt. Modulvor- und -rücklauf 1r und 1s bestehen jeweils aus einem Rohr mit Anschlußstücken 1t für die Verbinder 1q, wobei die Querschnittsfläche des Modulvorlaufs 1r in Fließrichtung abnimmt und die Querschnittsfläche des Modulrücklaufs 1s in Fließrichtung zunimmt.
Durch die querschnittsreduzierten bzw. -erweiterten Verbinder 1q und die querschnittsreduzierten bzw. -erweiterten Modulvor- und -rückläufe wird ein gleichmäßiges Strömungsprofil über alle Dünnschichtgefäße 1o ermöglicht. Die Dünnschichtgefäße 1o sind außerdem mit einer Vielzahl von Konditionierungsturbulatoren ausgestattet, welche auch bei geringer Fließgeschwindigkeit turbulente Strömungsverhältnisse gewährleisten und das Oberflächen-/Grundflächenverhältnis erhöhen sowie das einfallende Licht im Dünnschichtgefäß optimal verteilen und auftretende Immobilisierungen an den Gefäßoberflächen von Reinigungskörpern in der Suspension verhindern bzw. abbauen.

Das Dünnschichtsystem 1a wird im vorliegenden Fall aus 4 Glasrohrmodulen gebildet, welche jeweils aus einem Gestell 1v mit beidseitig versetzt zueinander angebrachten Rohrhalterungen bestehen, in welchen die Rohre 1o übereinander und jeweils versetzt zueinander angeordnet sind.

Die Oberfläche und/oder das Wandmaterial der Glasrohre 10 und/oder das Gestell 1v bzw. die Rohrhalterungen (nicht dargestellt) und/oder die Grundfläche, auf der das Dünnschichtsystem 1a angeordnet ist, sind mit Mitteln, insbesondere aus UV-fluoreszierendem und/oder transparentem und/oder reflektierendem Material zur quantitativen und qualitativen Veränderung der Reaktorgefäßbelichtung 1h und 1i versehen. Dadurch wird die zur Verfügung stehende Strahlungsmenge im photosynthetisch aktiven Bereich für die Belichtung der Mikroorganismen in den Dünnschichtgefäßen erhöht. Die Mittel können aber auch aus IR-reflektierendem und/oder transparentem Material bestehen, wodurch eine Erhöhung der Suspensionstemperatur verhindert und eine Strahlungskühlung der Kultursuspension durch das Wandmaterial des transparenten Dünnschichtgefäßes gewährleistet wird. Im Verlaufe des Wachstums der Biomasse im Dünnschichtsystem 1a werden energiereiche Kohlenstoffverbindungen aufgebaut. Die Biomasseemte erfolgt bei einer Trockenmassekonzentration in der Kultursuspension von > 2 g/l.

Mit Hilfe der Systempumpe 1f wird die Kultursuspension dazu der Emtevorrichtung 2 zugeführt. Diese besteht aus einem Emtebehälter 2a, einer Emtepumpe 2b, einem Separator 2c und einem Biomassebehälter 2d.
Der Separator 2c trennt die Biomasse von der Nährlösung, welche zur Nährlösungsaufbereitungsvorrichtung 4 und/oder direkt zur Nährlösungs- bzw. Stammlösungsversorgungseinrichtung 3 zurückgeführt wird.
Die Vorrichtung 4 besteht aus einem mechanischen Vorfilter (nicht dargestellt) und einem Aktivkohlefilter 4a, welcher kontaminierte Stoffe filtert und Nährstoffe für die Mikroalgen ungehindert durchströmen läßt.
Die somit erhaltene Biomasse wird einer Verarbeitungsvorrichtung (nicht dargestellt) zugeführt, wobei energiereiche Kohlenstoffverbindungen als Energieträger zur Produktion von chemischen und/oder pharmazeutischen Grund- und Wirkstoffen und/oder zur Produktion von hochwertigen Futtermitteln bzw. Futtermittelzusätzen gewonnen werden.

## Patentansprüche

1. Anlage zur Produktion von phototrophen Organismen und Zellkulturen unter Lichteinwirkung durch photochemische und photokatalytische Reaktionen und photoinduzierbare Prozesse, bestehend aus einem Bioreaktor (1), einer Emtevorrichtung (2), einer Nährlösungs- bzw. Stammlösungsversorgungseinrichtung (3) und einer Nährlösungsaufbereitungsanlage (4), wobei der Bioreaktor (1) ein photosynthetisch und hydrodynamisch aktives Dünnschichtsystem (1a), ein Vor- und Rücklaufsystem (1b, 1c), einen Konditionierbehälter (1d), einen Wärmetauscher (1e), eine Systempumpe (1f), eine Meßstrecke (1g), Mittel zur quantitativen und qualitativen Veränderung der Reaktorgefäßbelichtung (1h, 1i) und Mittel zur Gaszu- bzw. -ableitung aufweist, **dadurch gekennzeichnet, daß**
das photosynthetisch und hydrodynamisch aktive Dünnschichtsystem (1a) aus mindestens einem Modul mit einer Vielzahl von zueinander beabstandeten, übereinanderliegenden und parallel zueinander verlaufenden röhrenförmigen Dünnschichtgefäßen (1a) besteht, deren Eintritts- und Austrittsseiten über Anschlußstücke (1p, 1t) und Verbinder (1q) mit einem Modulvorlauf (1r) bzw. -rücklauf (1s) verbunden sind, wobei die einzelnen Verbinder (1q) für die übereinanderliegenden Dünnschichtgefäße (1o) jeweils eine Querschnittsfläche aufweisen, die ein gleichmäßiges Strömungsprofil über alle Dünnschichtgefäße ermöglicht.

2. Anlage nach Anspruch 1, **dadurch gekennzeichnet, daß**
die Querschnittsfläche der Verbinder (1q) in Fließrichtung des Modulvorlaufes (1r) zunimmt und in Fließrichtung des Modulrücklaufes (1s) abnimmt.

3. Anlage nach einem der obigen Ansprüche,
**dadurch gekennzeichnet, daß**
Modulvor- und -rücklauf (1r, 1s) jeweils aus einem Rohr mit Anschlußstücken (1t) für die Verbinder (1q) bestehen, wobei die Querschnittsfläche des Modulvorlaufs (1r) in Fließrichtung abnimmt und die Querschnittsfläche des Modulrücklaufs (1s) in Fließrichtung zunimmt.

4. Anlage nach einem der obigen Ansprüche,
**dadurch gekennzeichnet, daß**
die Dünnschichtgefäße (1o) eine Vielzahl von Konditionierungsturbulatoren aufweisen.

5. Anlage nach einem der obigen Ansprüche,
**dadurch gekennzeichnet, daß**
das Dünnschichtsystem (1a) aus mindestens einem Glasrohrmodul besteht, welches ein Gestell (1v) mit beidseitig versetzt zueinander angebrachten Rohrhalterungen umfaßt, in welchem die Rohre (1o) übereinander und jeweils versetzt zueinander angeordnet sind.

6. Anlage nach einem der obigen Ansprüche,
**dadurch gekennzeichnet, daß**
die Oberfläche und/oder das Wandmaterial der Dünnschichtgefäße und/oder das Gestell (1v) bzw. die Rohrhalterungen des Glasrohrmoduls und/oder die Grundfläche, auf der die Glasrohrmodule angeordnet sind und/oder zwischen den Rohren Mittel zur quantitativen und qualitativen Veränderung der Raktorgefäßbelichtung (1h, 1i) aufweisen.

7. Anlage nach einem der obigen Ansprüche,
**dadurch gekennzeichnet, daß**
die Mittel zur quantitativen und qualitativen Veränderung der Reaktorgefäßbelichtung (1h, 1i) aus UV-fluoreszierenden und/oder reflektierenden und/oder antireflektierenden und/oder transparentem Material bestehen.

8. Anlage nach einem der obigen Ansprüche,
**dadurch gekennzeichnet, daß**
die Mittel zur quantitativen und qualitativen Veränderung der Reaktorgefäßbelichtung (1h, 1i) aus IR-reflektierendem und/oder transparentem Material bestehen.

9. Anlage nach einem der obigen Ansprüche,
**dadurch gekennzeichnet, daß**
die Mittel zur Gaszu- bzw. -ableitung einen im oder am Boden der Anlage angeordneten temperierbaren Rücklaufsammler (1j), eine Vorrichtung zur CO₂-Einbindung in die Kultursuspension, eine Vorrichtung zum O₂-Austrag (1k) aus der Kultursuspension und einen Gasverteiler (1I) im Rücklaufsammler (1j) umfassen.

10. Anlage nach einem der obigen Ansprüche,
**dadurch gekennzeichnet, daß**
der Konditionierbehälter (1d), der Wärmetauscher (1e) und die Mittel zur Gaszu- bzw. -ableitung miteinander verbunden sind, wobei der Rücklaufsammler (1j) gleichzeitig einen Teil des Wärmetauschers (1e) und/oder des Konditionierbehälters (1d) bildet.

11. Anlage nach einem der obigen Ansprüche,
**dadurch gekennzeichnet, daß**
die Erntevorrichtung (2) aus einem Erntebehälter (2a), einer Emtepumpe (2b), einem Separator (2c) und einem Biomassebehälter (2d) besteht.

12. Anlage nach einem der obigen Ansprüche,
**dadurch gekennzeichnet, daß**
die Nährlösungs- bzw. Stammlösungsversorgungsvorrichtung (3) eine Nährlösungspumpe, einen Stammlösungsbehälter mit Rührwerk und einen Nährlösungsbehälter umfaßt.

13. Anlage nach einem der obigen Ansprüche,
**dadurch gekennzeichnet, daß**
die Nährlösungsaufbereitungsvorrichtung (4) aus einem mechanischen Vorfilter und einem Aktivkohlefilter (4a) besteht und mit der Erntevorrichtung (2) und/oder Nährlösungs- bzw. Stammlösungsversorgungsvorrichtung (3) verbunden ist.

## Claims

1. Apparatus for producing phototrophic organisms and cell cultures by the effect of light using photochemical and photocatalytic reactions and photo-inducible processes, comprising a bioreactor (1), a harvesting device (2), an arrangement (3) for supplying nutrient solution or stock solution, and a nutrient solution processing apparatus (4), the bioreactor (1) having a photosynthetically and hydrodynamically active thin-layer system (1a), an advancing and a returning system (1b, 1c), a treatment container (1d), a heat exchanger (1e), a system pump (1f), a measurement path (1g), means for quantitatively and qualitatively changing the reactor vessel illumination (1h, li), and means for feeding or discharging gas, **characterised in that** the photosynthetically and hydrodynamically active thin-layer system (1a) comprises at least one module having a plurality of spaced-apart, tubular thin-layer vessels (1), which lie one above the other and extend parallel to one another, the inlet and outlet sides of which vessels are connected to a module advancing means (1r) or returning means (1s) via connection pieces (1p, 1t) and connectors (1q), the individual connectors (1q) for the thin-layer vessels (1o), which lie one above the other, each having a cross-sectional area which permits a uniform flow profile over all of the thin-layer vessels.

2. Apparatus according to claim 1, **characterised in that** the cross-sectional area of the connectors (1) increases when viewed with respect to the flow direction of the module advancing means (1r), and it decreases when viewed with respect to the flow direction of the module returning means (1s).

3. Apparatus according to one of the above claims, **characterised in that** the module advancing and returning means (1r, 1s) each comprise a tube having connection pieces (1p, 1t) for the connectors (1q), the cross-sectional area of the module advancing means (1r) decreasing when viewed with respect to the flow direction, and the cross-sectional area of the module returning means (1s) increasing when viewed with respect to the flow direction.

4. Apparatus according to one of the above claims, **characterised in that** the thin-layer vessels (1o) have a plurality of treatment turbulators (lu).

5. Apparatus according to one of the above claims, **characterised in that** the thin-layer system (1a) comprises at least one glass-tube module, which includes a support (1v) having tube holders mounted so as to be offset from one another on each side, and in which module the tubes (1o) are disposed above one another and offset from one another.

6. Apparatus according to one of the above claims, **characterised in that** the surface and/or the wall material of the thin-layer vessels and/or the support (1v) or the tube holders for the glass-tube module and/or the bottom face, on which the glass-tube modules are disposed, and/or between the tubes have means for quantitatively and qualitatively changing the reactor vessel illumination (1h, 1i).

7. Apparatus according to one of the above claims, **characterised in that** the means for quantitatively and qualitatively changing the reactor vessel illumination (1h, 1i) comprise UV-fluorescent and/or reflecting and/or anti-reflecting and/or transparent material.

8. Apparatus according to one of the above claims, **characterised in that** the means for quantitatively and qualitatively changing the reactor vessel illumination (1h, 1i) comprise IR-reflecting material and/or transparent material.

9. Apparatus according to one of the above claims, **characterised in that** the means for feeding or discharging gas include a temperature-controllable return accumulator (1j) disposed in or on the base of the apparatus, a device for incorporating CO₂ into the culture suspension, a device (1k) for removing O₂ from the culture suspension, and a gas distributor (11) in the return accumulator (1j).

10. Apparatus according to one of the above claims, **characterised in that** the treatment container (1d), the heat exchanger (1e) and the means for feeding or discharging gas are interconnected, the return accumulator (1j) simultaneously forming a part of the heat exchanger (1e) and/or of the treatment container (1d).

11. Apparatus according to one of the above claims, **characterised in that** the harvesting device (2) comprises a harvesting container (2a), a harvesting pump (2b), a separator (2c) and a biomass container (2d).

12. Apparatus according to one of the above claims, **characterised in that** the arrangement (3) for supplying nutrient solution or stock solution includes a nutrient solution pump (3a), a stock solution container (3b) with an agitator, and a nutrient solution container (3c).

13. Apparatus according to one of the above claims, **characterised in that** the nutrient solution processing device (4) comprises a mechanical preliminary filter and an active carbon filter (4a) and is connected to the harvesting device (2) and/or arrangement (3) for supplying nutrient solution or stock solution.

## Revendications

1. Installation de production d'organismes phototrophes et de cultures cellulaires sous l'effet de la lumière par des réactions photochimiques et photocatalytiques et par des processus photoinductifs, composée d'un bioréacteur (1), d'un dispositif de récolte (2), d'un dispositif d'alimentation en bouillon de culture ou liqueur mère (3), et d'une installation de traitement du bouillon de culture (4), le bioréacteur (1) présentant un système à couche mince (1a) à activité photosynthétique et hydrodynamique, un système aller et retour (1b, 1c), un réservoir de conditionnement (1d), un échangeur thermique (1e), une pompe de système (1f), une section de mesure (1g), des moyens pour la modification quantitative et qualitative de l'exposition lumineuse du réacteur (1h, 1i), et des moyens pour l'arrivée et l'évacuation de gaz, **caractérisée en ce que** le système à couche mince (1a) à activité photosynthétique et hydrodynamique se compose d'au moins un module avec une multiplicité de récipients à couche mince (1o) tubulaires, distants les uns des autres, superposés et parallèles entre eux, dont les côtés entrée et sortie sont reliés respectivement à un aller (1r) et retour (1s) de module par l'intermédiaire de pièces de jonction (1p, 1t) et de raccords (1q), les raccords individuels (1q) pour les récipients à couche mince (1o) superposés présentant chacun une section transversale qui permet un profil d'écoulement régulier sur tous les récipients à couche mince.

2. Installation suivant la revendication 1, **caractérisée en ce que** la section transversale des raccords (1q) augmente dans le sens d'écoulement de l'aller de module (1r) et diminue dans le sens d'écoulement du retour de module (1s).

3. Installation suivant l'une des revendications précédentes, **caractérisée en ce que** l'aller et le retour de module (1r, 1s) se composent chacun d'un tube avec des pièces de jonction (1p, 1t) pour les raccords (1q), la section transversale de l'aller de module (1r) diminuant dans le sens d'écoulement, et la section transversale du rétour de module (1s) augmentant dans le sens d'écoulement.

4. Installation suivant l'une des revendications précédentes, **caractérisée en ce que** les récipients à couche mince (1o) présentent une multiplicité de turbulateurs de conditionnement (1u).

5. Installation suivant l'une des revendications précédentes, **caractérisée en ce que** le système à couche mince (1a) se compose d'au moins un module de tubes de verre, qui comprend un support (1v) avec des fixations de tubes montées de part et d'autre en déport mutuel, dans lequel les tubes (1o) sont superposés et respectivement disposés en déport mutuel.

6. Installation suivant l'une des revendications précédentes, **caractérisée en ce que** la surface et/ou le matériau de paroi des récipients à couche mince et/ou le support (1v) ou les fixations de tubes du module de tubes de verre et/ou la surface de base, sur laquelle sont disposés les modules de tubes de verre et/ou entre les tubes, présentent des moyens pour la modification quantitative et qualitative de l'exposition lumineuse (1h, 1i) du réacteur.

7. Installation suivant l'une des revendications précédentes, **caractérisée en ce que** les moyens pour la modification quantitative et qualitative de l'exposition lumineuse (1h, 1i) du réacteur se composent de matériau fluorescent UV et/ou réfléchissant et/ou anti-réfléchissant et/ou transparent.

8. Installation suivant l'une des revendications précédentes, **caractérisée en ce que** les moyens pour la modification quantitative et qualitative de l'exposition lumineuse (1h, 1i) du réacteur se composent de matériau réfléchissant IR et/ou transparent.

9. Installation suivant l'une des revendications précédentes, **caractérisée en ce que** les moyens pour l'arrivée et l'évacuation de gaz comprennent un collecteur de retour (1j) pouvant être équilibré en température, disposé dans ou sur le fond de l'installation, un dispositif pour l'intégration CO₂ dans la suspension de culture, un dispositif pour la décharge O₂ (1k) de la suspension de culture, et un distributeur de gaz (1I) dans le collecteur de retour (1j).

10. Installation suivant l'une des revendications précédentes, **caractérisée en ce que** le réservoir de conditionnement (1d), l'échangeur thermique (1e), et les moyens pour l'arrivée et l'évacuation de gaz, sont reliés entre eux, le collecteur de retour (1j) formant simultanément une partie de l'échangeur thermique (1e) et/ou du réservoir de conditionnement (1d).

11. Installation suivant l'une des revendications précédentes, **caractérisée en ce que** le dispositif de récolte (2) se compose d'un réservoir de récolte (2a), d'une pompe à récolte (2b), d'un séparateur (2c) et d'un. réservoir de biomasse (2d).

12. Installation suivant l'une des revendications précédentes, **caractérisée en ce que** le dispositif d'alimentation en bouillon de culture ou liqueur mère (3) comprend une pompe à bouillon de culture (3a), un réservoir de liqueur mère (3b) avec agitateur, et un réservoir de bouillon de culture (3c).

13. Installation suivant l'une des revendications précédentes, **caractérisée en ce que** le dispositif de traitement de bouillon de culture (4) se compose d'un pré-filtre mécanique et d'un filtre à charbon actif (4a), et est relié au dispositif de récolte (2) et/ou au dispositif d'alimentation en bouillon de culture ou liqueur mère (3).
